# FASCICULE DE BREVET EUROPEEN

(11) **EP 2 670 467 B1**
(45) Date de publication et mention de la délivrance du brevet: **22.03.2017**
(21) Numéro de dépôt: 12705346.0
(22) Date de dépôt: 20.01.2012
(51) Int. Cl.: A61M 16/12, A61M 16/04, A61M 16/06

(54) **DISPOSITIF DE RESPIRATION ARTIFICIELLE POUR LA RÉANIMATION D'UNE PERSONNE EN ÉTAT D'ARRÊT CARDIAQUE**
KÜNSTLICHE BEATMUNGSVORRICHTUNG ZUR WIEDERBELEBUNG EINER PERSON MIT HERZSTILLSTAND
ARTIFICIAL RESPIRATION DEVICE FOR RESUSCITATING A PERSON IN A STATE OF CARDIAC ARREST

(30) Priorité: 03.02.2011 FR 1100338
(43) Date de publication de la demande: 11.12.2013
(73) Titulaire: Boussignac, Georges, 92160 Antony (FR)
(72) Inventeur: Boussignac, Georges, 92160 Antony (FR)
(74) Mandataire: Jacobacci Coralis Harle
(86) Numéro de dépôt international: PCT/FR2012/050124
(87) Numéro de publication internationale: WO 2012/104517

(56) Documents cités:
- EP-A1- 0 701 834
- EP-A1- 0 911 051
- EP-A1- 2 228 088
- FR-A1- 2 911 073
- FR-A1- 2 912 660
- FR-A1- 2 921 840

## Description

La présente invention concerne un dispositif de respiration artificielle utilisable pendant la réanimation de personnes en état d'arrêt cardiaque.

On sait que, pour tenter de réanimer une personne en état d'arrêt cardiaque, on exerce sur la cage thoracique de cette personne des compressions et des décompressions rythmées alternées tendant à rétablir les mouvements d'expiration et d'inspiration, ainsi que la circulation sanguine.

Par ailleurs, par la demande européenne EP-0911051 (propriété du Demandeur), on connaît déjà un dispositif de respiration artificielle pour des personnes en état d'arrêt cardiaque et en cours de réanimation par compressions et décompressions alternées de leur cage thoracique. Un tel dispositif de respiration artificielle connu comporte :
- un élément tubulaire qui forme un canal principal et qui est destiné à être relié par son extrémité distale à une voie respiratoire d'une personne, alors que l'extrémité proximale dudit élément tubulaire se trouve à l'extérieur de ladite personne et que le système respiratoire de celui-ci est relié à l'extérieur par l'intermédiaire dudit canal principal ;
- des canaux auxiliaires périphériques qui débouchent dans ledit canal principal, lesdits canaux auxiliaires étant alimentés en continu en gaz respiratoire ; et
- des moyens de déflexion, pour faire converger les uns vers les autres, à l'intérieur dudit canal principal, les jets de gaz respiratoire injectés en continu par lesdits canaux auxiliaires.

Ainsi, le patient est ventilé en continu par lesdits jets de gaz respiratoire.

Le demandeur a trouvé que ce dispositif d'assistance respiratoire pour patient à respiration spontanée peut être utilisé avec succès comme dispositif de respiration artificielle (et non plus seulement comme dispositif d'assistance respiratoire) sur des personnes en état d'arrêt cardiaque et en cours de réanimation par compressions et décompressions alternées de leur cage thoracique, les jets dudit gaz respiratoire favorisant la reprise de l'inspiration et de la circulation sanguine.

Cependant, le Demandeur a remarqué que ledit gaz respiratoire, introduit en continu dans les poumons de la personne en état d'arrêt cardiaque, engendre dans ceux-ci, à la fin d'une compression et au début de la décompression suivante, une pression résiduelle positive, qui se maintient pendant une partie de ladite décompression, avant de disparaître et d'être remplacée par une pression négative engendrée par la décompression. Une telle pression résiduelle positive, d'une part, forme un obstacle à l'aspiration d'air extérieur à travers ledit élément tubulaire et, d'autre part, est entretenue par ledit air extérieur aspiré. Il en résulte que, pendant une partie importante de chaque décompression, les poumons de ladite personne aspirent mal l'air extérieur et que la circulation sanguine (notamment le retour veineux) n'est pas assurée de façon satisfaisante aux extrémités (tête, bras, jambes) de ladite personne.

On connaît par FR 2 912 660 A1 un dispositif de respiration artificielle comportant un élément tubulaire et une soupape comportant une membrane élastique montée sur une bague interne saillante. On connaît par EP 2 228 088 A1 un dispositif de respiration artificielle à élément tubulaire comportant une bague interne au niveau d'une zone de pression crée par des jets de gaz. La présente invention a pour objet de remédier à cet inconvénient de la pression résiduelle positive due aux jets de gaz respiratoire spécifié ci-dessus.

L'invention propose un dispositif de respiration artificielle utilisable pour la réanimation d'une personne en arrêt cardiaque, dont la cage thoracique est soumise à des compressions et décompressions alternées tendant à rétablir les mouvements d'expiration et d'inspiration, ledit dispositif comportant :
- un élément tubulaire qui forme un canal principal et qui est destiné à être relié par son extrémité distale à une voie respiratoire de ladite personne, alors que l'extrémité proximale dudit élément tubulaire se trouve à l'extérieur de ladite personne et que le système respiratoire de celle-ci est relié à l'extérieur par l'intermédiaire dudit canal principal;
- des canaux auxiliaires périphériques qui débouchent dans ledit canal principal, lesdits canaux auxiliaires étant alimentés en continu en gaz respiratoire; et
- des moyens de déflexion, pour faire converger les uns vers les autres, à l'intérieur dudit canal principal, les jets de gaz respiratoire injectés en continu par lesdits canaux auxiliaires, de sorte qu'une zone de pression de forme oblongue prend naissance à l'extrémité distale desdits canaux auxiliaires et s'allonge dans la direction distale le long de l'axe longitudinal dudit canal principal;
- lequel dispositif comporte au moins une restriction locale de section fixe présentant une forme et une disposition constantes, de manière à former une résistance locale fixe sur l'écoulement gazeux traversant ledit canal principal quel que soit son sens d'écoulement; et ladite restriction locale, qui se présente sous la forme d'une bague interne saillante, est longitudinalement éloignée et à l'extérieur de ladite zone de pression oblongue, et ladite restriction locale est agencée en amont, c'est-à-dire côté proximal, de ladite zone de pression oblongue.

Ainsi, grâce à l'invention, lors d'une compression de la cage thoracique de la personne en cours de réanimation, la résistance locale, apparaissant au niveau de ladite restriction et s'exerçant sur l'écoulement gazeux traversant le canal principal, engendre une augmentation de pression (pression positive) à l'intérieur des poumons, l'air chassé desdits poumons s'échappant librement mais plus difficilement qu'en l'absence de restriction locale.

Inversement, lors d'une décompression, la pression baisse davantage (pression négative) dans les poumons qu'avec un dispositif de respiration artificielle connu dépourvu de restriction locale. Le freinage de l'entrée d'air extérieur, engendré par ladite restriction, permet une aspiration progressive et contrôlée de l'air extérieur en direction des poumons de la personne, ce qui entraîne la disparition, au début de la décompression, de la pression résiduelle positive due aux jets de gaz respiratoire.

La pression résiduelle positive disparaît rapidement sous l'action de la décompression, pendant l'entrée progressive de l'air extérieur aspiré. La pression résiduelle positive ne constitue donc plus un obstacle à l'aspiration d'air extérieur et à la circulation sanguine de la personne en arrêt cardiaque.

La variation de pression intrathoracique entre une compression et une décompression, obtenue selon l'invention, est étendue en comparaison des variations de pression intrathoracique observées sur des personnes en cours de réanimation équipées d'un dispositif de respiration artificielle connu, par exemple du type de celui décrit par la demande de brevet EP-091 1051. La surface d'échange gazeux est ainsi augmentée et le retour veineux amélioré. Ces phénomènes s'expliquent notamment par l'application de la physique des corps inertes au corps de la personne en cours de réanimation. A titre d'exemple illustratif, la pression intrathoracique peut atteindre 15 cm d'eau, lors d'une compression, et -7 cm d'eau, lors d'une décompression.

De plus, la restriction locale est dépourvue d'élément mobile (ce qui en simplifie la fabrication) et présente une forme et une disposition constantes qu'une compression ou qu'une décompression soit exercée sur la cage thoracique de la personne en état d'arrêt cardiaque. Autrement dit, la restriction locale forme un moyen de freinage de gaz statique et passif, dépourvu d'inertie.

De plus, le dispositif de l'invention, une fois relié au système respiratoire de la personne en état d'arrêt cardiaque, forme un système ouvert qui prévient l'apparition de surpression dans l'estomac (autrement dit, on évite tout « gonflage de l'estomac ») et permet une alimentation continue en gaz respiratoire tout en appliquant, sans interruption, des compressions et des décompressions alternées sur la cage thoracique de ladite personne. On réduit le risque de traumatisme du système respiratoire de cette dernière, tout en améliorant l'hémodynamique.

Ladite restriction locale est agencée en amont, c'est-à-dire du côté proximal, de ladite zone de pression oblongue.

Lorsque le dispositif de l'invention comporte deux restrictions locales, celles-ci peuvent être disposées en aval et en amont, respectivement, de ladite zone de pression oblongue.

De préférence, ladite restriction locale forme une restriction de la section interne de l'élément tubulaire et fait partie intégrante dudit dispositif.

En variante, le dispositif de l'invention peut comporter un corps tubulaire amovible, dans lequel est disposée ladite restriction locale formant une restriction de la section interne dudit corps tubulaire.

En outre, le dispositif de l'invention peut comporter des moyens d'aspiration d'air ambiant, par exemple du type à effet Venturi, qui sont entrainés par du gaz circulant dans ledit canal principal.

La présente invention concerne également un masque laryngé comprenant un dispositif de respiration artificielle du type de celui spécifié ci-dessus.

Les figures du dessin annexé feront bien comprendre comment l'invention peut être réalisée. Sur ces figures, des références identiques désignent des éléments semblables.
La figure 1 est une vue schématique, partiellement en coupe axiale, d'un masque respiratoire bucco-nasal équipé d'un exemple de réalisation d'un dispositif de respiration artificielle utilisable dans le cadre de la présente invention.
Les figures 2 et 3 sont des coupes transversales du dispositif de respiration artificielle de la figure 1, respectivement selon les lignes II-II et III-III.
La figure 4 est, une coupe longitudinale schématique, d'un masque laryngé pourvu d'un exemple de réalisation d'un dispositif de respiration artificielle conforme à l'invention.
La figure 5 illustre schématiquement en coupe une variante de réalisation des exemples des figures 1 et 4 d'un dispositif conforme à la présente invention.
La figure 6 est vue schématique selon la flèche VI du corps tubulaire amovible du dispositif conforme à l'invention de la figure 5.

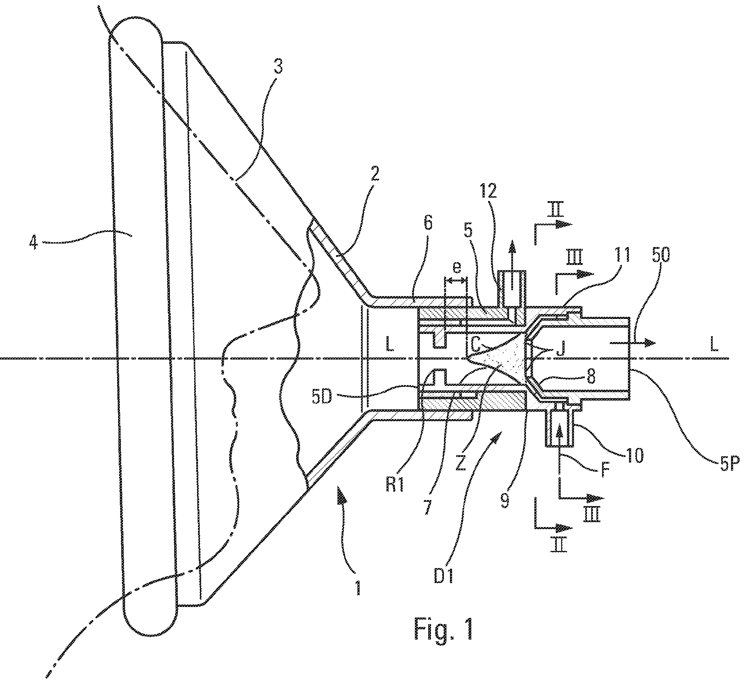

Le masque respiratoire bucco-nasal 1, représenté sur la figure 1, comporte une coque rigide de forme générale tronconique 2, pouvant être appliquée sur le visage d'une personne 3 par l'intermédiaire d'un coussinet 4, bordant son ouverture périphérique. Du côté opposé, ledit masque 1 est pourvu d'un dispositif de respiration artificielle D1 comportant un élément tubulaire rigide 5, solidaire de ladite coque 2 ou emboîté sur une saillie tubulaire 6 de celle-ci. L'élément tubulaire 5 sert d'embout d'entrée et de sortie de gaz dans le masque 1, son extrémité proximale 5P étant du côté de l'air libre, alors que son extrémité distale 5D se trouve du côté du masque 1.

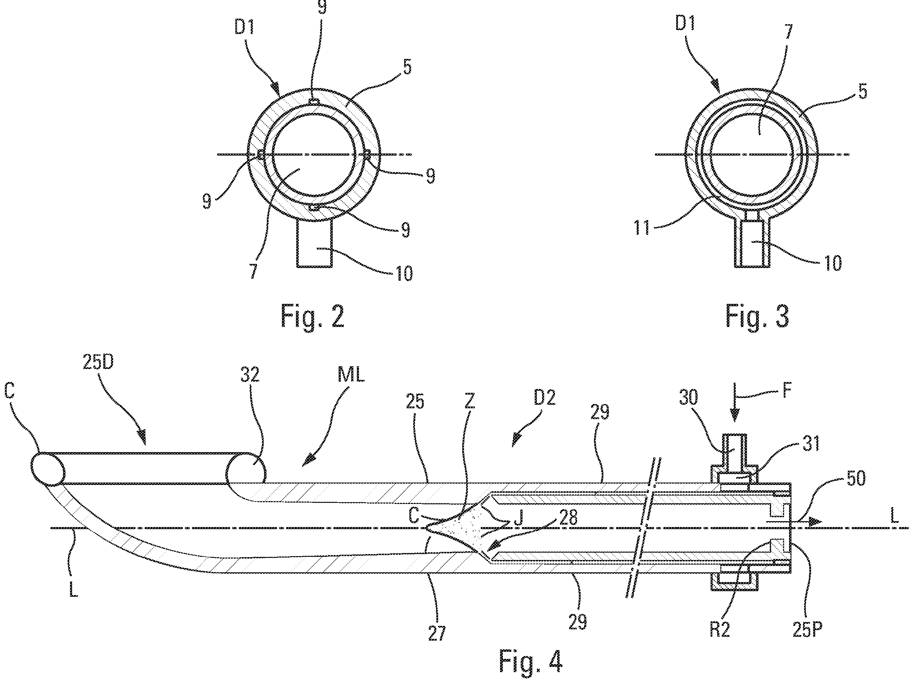

L'élément tubulaire 5 forme un canal principal interne 7 et il comporte, en partie médiane, des moyens de déflexion 8, dirigés vers l'axe longitudinal L-L dudit canal 7. Les moyens de déflexion 8 ont pour objet de défléchir, en direction dudit axe du canal principal 7, des jets de gaz respiratoire J injectés par des canaux auxiliaires périphériques 9, alimentés à partir d'un embout d'amenée 10 (voir la flèche F symbolisant l'alimentation en gaz respiratoire), par l'intermédiaire d'une chambre annulaire périphérique 11. Lesdits jets de gaz respiratoire convergent ainsi vers un point de convergence C de l'axe L-L dudit canal principal 7 (voir également les figures 2 et 3). Il se forme une zone de pression Z de forme oblongue qui prend naissance à l'extrémité distale desdits canaux auxiliaires 9 et s'allonge dans la direction distale le long de l'axe longitudinal L-L du canal principal 7.

Par ailleurs, l'élément tubulaire 5 comporte un embout 12 pour le prélèvement de gaz et/ou la mesure de pression.

Dans l'exemple de réalisation illustrée en figure 4, le masque laryngé ML est équipé d'un dispositif de respiration artificielle D2 comportant un élément tubulaire 25 souple, dont l'extrémité distale 25D est destinée à être introduite dans une voie respiratoire d'un patient, alors que l'extrémité proximale 25P de l'élément 25 reste extérieure audit patient. L'élément tubulaire 25 forme un canal principal interne 27 et il comporte des moyens de déflexion 28, dirigés vers l'axe L-L dudit canal 27. Les moyens de déflexion 28 ont pour objet de défléchir, en direction dudit axe L-L, des jets de gaz respiratoire J injectés par des canaux auxiliaires périphériques 29, alimentés à partir d'un conduit d'alimentation 30 (voir la flèche F), par l'intermédiaire d'une chambre périphérique 31, lesdits jets de gaz respiratoire J convergeant vers un point de convergence C dudit axe L-L. De la même manière que pour D1, il se forme une zone de pression Z de forme oblongue.

Le masque laryngé ML comprend un coussinet 32 en silicone à ballonnet gonflable qui borde le contour l'extrémité distale ouverte 25D.

Notamment lorsqu'ils sont utilisés pour la réanimation d'urgence de personnes en arrêt cardiaque, dont la cage thoracique est soumise à des compressions et décompressions alternées, les dispositifs de respiration artificielle D1 ou D2 sont alimentés en continu en gaz respiratoire (flèche F) à partir de bouteilles de gaz sous pression ou analogues et les jets J sont continus pendant tout le processus de réanimation. De façon générale, les bouteilles de gaz à usage médical délivrent leur gaz sous une pression nominale de l'ordre de 3,5 bars (3,5 x 10⁵ pascals), avec un débit dont les variations sont bornées et connues.

Chacun des dispositifs D1 et D2 comporte une restriction locale R1, R2 de la section interne de l'élément tubulaire 5, 25, qui se présente sous la forme d'une bague interne saillante rigide disposée à l'intérieur du canal principal 7, 27. Une telle restriction locale R1, R2 fait partie intégrante du dispositif D1, D2 correspondant.

Dans l'exemple de la figure 1, la restriction locale R1 de la section interne du canal principal 7 du dispositif D1 est agencée en aval de la zone de pression oblongue Z, à l'intérieur dudit canal principal 7.

Cette restriction locale R1 est longitudinalement éloignée et à l'extérieur de la zone de pression Z. Il existe un écartement e entre l'extrémité distale de la zone de pression Z et le plan transversal à l'axe L-L dans lequel est agencée la restriction R1.

La fabrication du dispositif D1 de se fait, par exemple, en tenant compte des paramètres (pression, débit) propres aux bouteilles de gaz ou analogues, pour que la restriction locale de section R1 soit éloignée et à l'extérieur de la zone de pression oblongue Z.

Dans l'exemple de réalisation de la présente invention illustré par la figure 4, la restriction locale R2 de la section interne du canal principal 7 de D2 est positionnée en amont de la zone de pression oblongue Z, à l'intérieur dudit canal principal 27.

Plus précisément, la restriction locale R2 est située au voisinage de l'extrémité proximale 25P de l'élément tubulaire 25, de telle façon qu'elle se retrouve éloignée et distante longitudinalement de la zone de pression Z.

Lors d'une compression de la cage thoracique de la personne 3 équipée du dispositif D1, D2, la résistance locale occasionnée par chacune des restrictions R1, R2 provoque une augmentation de pression dans les poumons de la personne 3, l'air chassé de ces derniers (symbolisé par la flèche 50 sur les figures 1 et 4) s'échappant librement mais plus difficilement.

Inversement, lors d'une décompression de la cage thoracique de ladite personne 3, la pression baisse davantage dans les poumons que lorsque les dispositifs D1 et D2 sont utilisés sans restriction locale, de sorte que la variation de pression intrathoracique entre une compression et une décompression augmente ce qui améliore l'effet de pompage de l'air extérieur. Le freinage de l'entrée d'air, lors d'une décompression, engendré par les restrictions R1 et R2 permet une aspiration progressive et contrôlée de l'air extérieur en direction des poumons de la personne, ce qui entraîne la disparition, au début de la décompression, de la pression résiduelle positive due aux jets J.

Que ce soit lors d'une compression ou d'une décompression, le canal principal 7, 27 reste ouvert.

De plus, dans le cas du masque bucco-nasal de la figure 1, la variation de pression au niveau de la bouche est quasi inexistante.

En outre, l'alimentation en gaz respiratoire (flèche F) des canaux auxiliaires périphériques 9, 29 des dispositifs D1, D2, à partir des embouts d'amenée 10, 30, peut être pilotée par l'intermédiaire d'une valve électronique (non représentée), par exemple commandée par un opérateur.

En particulier, une telle valve électronique permet, une fois le coeur et la circulation sanguine de nouveau en fonctionnement, de basculer d'une alimentation continue en gaz respiratoire des canaux auxiliaires 9, 29 à une alimentation impulsionnelle, de fréquence prédéfinie.

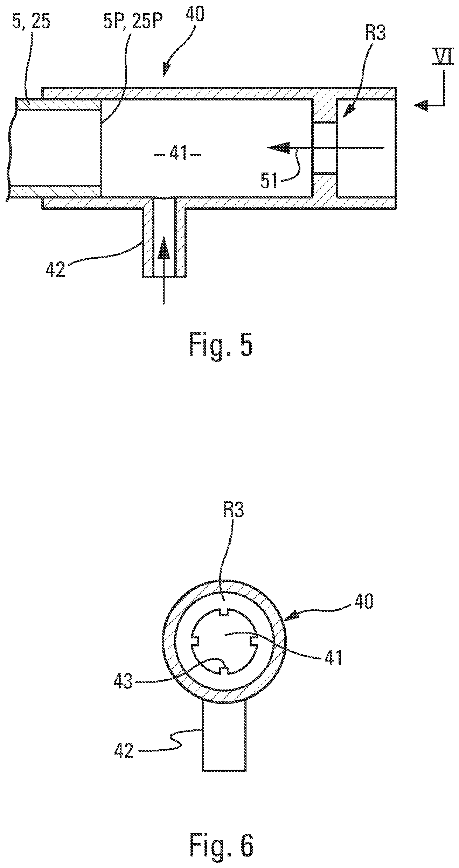

Par ailleurs, dans une variante de réalisation représentée sur la figure 5, chaque dispositif D1, D2 peut comporter un corps tubulaire 40 délimitant une cavité 41 en libre communication gazeuse avec l'élément tubulaire 5, 25. Le corps tubulaire 40 peut être rapporté, de façon amovible, au dispositif D1, D2 correspondant, à l'extrémité proximale 5P, 25P de l'élément tubulaire 5, 25 de celui-ci.

A l'intérieur du corps tubulaire 40 est disposée une restriction locale R3 de la section interne dudit corps 40.

On notera qu'un tel corps tubulaire 40 peut être rapporté à l'extrémité proximale des dispositifs D1, D2, que ces derniers comportent, ou non, une restriction locale de la section interne de l'élément tubulaire correspondant.

Par ailleurs, comme le montre schématiquement la figure 5, le corps tubulaire 40 comporte des moyens 42 d'aspiration d'air ambiant, du type à effet Venturi, qui sont entrainés par du gaz circulant dans le canal principal 7, 27 (par exemple de l'air extérieur, symbolisé par la flèche 51) lors d'une décompression de la cage thoracique de la personne en cours de réanimation.

En outre, comme cela est illustré par la figure 6, la bague interne saillante rigide R3, formant la restriction locale, comporte des obstacles 43, par exemple des ailettes convergentes, empêchant l'introduction accidentelle d'un objet apte à obturer hermétiquement le corps tubulaire 40. De tels obstacles 43 peuvent également être montés sur les restrictions R1 et R3.

De plus, le corps tubulaire 40 peut comporter un embout latéral saillant de raccord (non représenté sur les figures), en communication fluidique avec la cavité 41, pour raccorder un ballon insufflateur au corps tubulaire 40.

Il est à noter que le dispositif de la présente invention n'est pas exclusivement destiné à être mis en oeuvre avec un masque bucco-nasal ou un masque laryngé, mais pourrait, par exemple, être intégré à une sonde endotrachéale.

## Revendications

1. Dispositif de respiration artificielle utilisable pour la réanimation d'une personne en arrêt cardiaque, dont la cage thoracique est soumise à des compressions et décompressions alternées tendant à rétablir les mouvements d'expiration et d'inspiration, ledit dispositif comportant :
- un élément tubulaire (5, 25) qui forme un canal principal (7, 27) et qui est destiné à être relié par son extrémité distale (5D, 25D) à une voie respiratoire de ladite personne (3), alors que l'extrémité proximale (5P, 25P) dudit élément tubulaire se trouve à l'extérieur de ladite personne et que le système respiratoire de celle-ci est relié à l'extérieur par l'intermédiaire dudit canal principal (7, 27) ;
- des canaux auxiliaires (9, 29) périphériques qui débouchent dans ledit canal principal, lesdits canaux auxiliaires étant alimentés en continu en gaz respiratoire ; et
- des moyens de déflexion (8, 28), pour faire converger les uns vers les autres, à l'intérieur dudit canal principal (7, 27), les jets de gaz respiratoire (J) injectés en continu par lesdits canaux auxiliaires (9, 29), de sorte qu'une zone de pression de forme oblongue (Z) prend naissance à l'extrémité distale desdits canaux auxiliaires (9, 29) et s'allonge dans la direction distale le long de l'axe longitudinal (L-L) dudit canal principal, **caractérisé :**
- **en ce qu'**il comporte au moins une restriction locale de section fixe (R2, R3) présentant une forme et une disposition constantes, de manière à former une résistance locale fixe sur l'écoulement gazeux (50, 51) traversant ledit canal principal (7, 27) quel que soit son sens d'écoulement ; et
- **en ce que** ladite restriction locale, qui se présente sous la forme d'une bague interne saillante, est longitudinalement éloignée et à l'extérieur de ladite zone de pression oblongue (Z), et
**en ce que** ladite restriction locale (R2, R3) est agencée en amont, c'est-à-dire côté proximal, de ladite zone de pression oblongue (Z).

2. Dispositif de respiration artificielle selon la revendication 1, **caractérisé en ce que** ladite restriction locale (R2) forme une restriction de la section interne de l'élément tubulaire (25) et fait partie intégrante dudit dispositif (D2).

3. Dispositif de respiration artificielle selon la revendication 1, **caractérisé en ce qu'**il comporte un corps tubulaire (40) amovible, dans lequel est disposée ladite restriction locale (R3) formant une restriction de la section interne dudit corps tubulaire.

4. Dispositif de respiration artificielle selon l'une des revendications précédentes, **caractérisé en ce qu'**il comporte des moyens (42) d'aspiration d'air ambiant entraînés par du gaz circulant dans ledit canal principal (7, 27).

5. Dispositif de respiration artificielle selon l'une des revendications précédentes, **caractérisé en ce que** la bague interne saillante formant la restriction locale (R2, R3) comporte des obstacles (43) empêchant l'introduction accidentelle d'un objet qui serait apte à obturer hermétiquement l'élément ou le corps tubulaire.

6. Dispositif de respiration artificielle selon la revendication 5, **caractérisé en ce que** les obstacles (43) sont des ailettes convergentes.

7. Masque laryngé, **caractérisé en ce qu'**il comprend un dispositif de respiration artificielle (D2) tel que spécifié sous l'une des revendications précédentes.

## Patentansprüche

1. Vorrichtung für künstliche Beatmung zur Wiederbelebung einer Person mit Herzstillstand, deren Brustkorb abwechselnden Kompressions- und Entlastungsbewegungen unterzogen wird, um zu versuchen, die Einatmungs- und Ausatmungsbewegungen wiederherzustellen, wobei die Vorrichtung
- ein rohrförmiges Element (5, 25), das einen Hauptkanal (7, 27) bildet und das dazu bestimmt ist, mit seinem distalen Ende (5D, 25D) mit einem Atemweg der Person (3) verbunden zu werden, während sich das proximale Ende (5P, 25P) des rohrförmigen Elements außerhalb der Person befindet, wobei das Atemsystem derselben durch den Hauptkanal (7, 27) nach außerhalb verbunden ist,
- periphere Hilfskanäle (9, 29), die in den Hauptkanal münden, wobei die Hilfskanäle kontinuierlich mit Beatmungsgas versorgt werden, und
- Ablenkmittel (8, 28), um im Inneren des Hauptkanals (7, 27) die über die Hilfskanäle (9, 29) kontinuierlich eingespritzten Beatmungsgasstöße (J) aufeinander zu zu lenken, so daß eine Druckzone (Z) mit länglicher Form am distalen Ende der Hilfskanäle (9, 29) entsteht und sich in distaler Richtung entlang der Längsachse (L-L) des Hauptkanals erstreckt,
aufweist,
**dadurch gekennzeichnet, daß**
- sie wenigstens eine örtliche Verengung (R2, R3) festen Querschnitts aufweist, die eine konstante Form und eine konstante Anordnung aufweisen, um einen festen örtlichen Widerstand für den Gasfluß (50, 51) zu bilden, der, in welcher Strömungsrichtung auch immer, den Hauptkanal (7, 27) durchquert, und
- daß die örtliche Verengung, die in Form eines hervorstehenden inneren Rings vorliegt, in Längsrichtung entfernt und außerhalb der länglichen Druckzone (Z) liegt, und
daß die örtliche Verengung (R2, R3) stromaufwärts, d.h. auf der proximalen Seite der länglichen Druckzone (Z) liegt.

2. Vorrichtung für künstliche Beatmung gemäß Anspruch 1, **dadurch gekennzeichnet, daß** die örtliche Verengung (R2) eine Verengung des inneren Querschnitts des rohrförmigen Elements (25) bildet und integraler Bestandteil der Vorrichtung (D2) ist.

3. Vorrichtung für künstliche Beatmung gemäß Anspruch 1, **dadurch gekennzeichnet, daß** sie einen abnehmbaren rohrförmigen Körper (40) aufweist, in dem die eine Verengung des inneren Querschnitts des rohrförmigen Körpers bildende örtliche Verengung (R3) angeordnet ist.

4. Vorrichtung für künstliche Beatmung gemäß einem der vorangehenden Ansprüche, **dadurch gekennzeichnet, daß** sie Mittel (42) zum Ansaugen von Umgebungsluft aufweist, die durch das im Hauptkanal (7, 27) fließende Gas angetrieben werden.

5. Vorrichtung für künstliche Beatmung gemäß einem der vorangehenden Ansprüche, **dadurch gekennzeichnet, daß** der die örtliche Verengung (R2, R3) bildende hervorstehende innere Ring Hindernisse (43) aufweist, die ein ungewolltes Einbringen eines Gegenstands verhindern, der das rohrförmige Element oder den rohrförmigen Körper hermetisch verschließen könnte.

6. Vorrichtung für künstliche Beatmung gemäß Anspruch 5, **dadurch gekennzeichnet, daß** die Hindernisse (43) konvergierende Flügel sind.

7. Kehlkopfinaske, **dadurch gekennzeichnet, daß** sie eine Vorrichtung für künstliche Beatmung (D2), wie in einem der vorangehenden Ansprüche ausgeführt, aufweist.

## Claims

1. An artificial respiration device, in particular intended to the resuscitation of a person in cardiac arrest, whose thoracic cage is subjected to alternating compressions and decompressions tending to restore the expiration and inspiration movements, said device including:
- a tubular element (5, 25) that forms a main channel (7, 27) and that is intended to be connected by its distal end (5D, 25D) to a respiratory tract of said person (3), whereas the proximal end (5P, 25P) of said tubular element is external to said person and that the respiratory system of the latter is connected to the outside through said main channel (7, 27);
- peripheral auxiliary channels (9, 29) than open out into said main channel, said auxiliary channels being continuously supplied with respiratory gas; and
- deflection means (8, 28), for making converge toward each other, inside said main channel (7, 27), the jets of respiratory gas (J) continuously injected by said auxiliary channels (9, 29), so that an oblong-shaped pressure area (Z) originates at the distal end of said auxiliary channels (9, 29) and elongates in the distal direction, along the longitudinal axis (L-L) of said main channel,
**characterized:**
- **in that** it includes at least one local narrowing of constant section (R2, R3), of constant shape and arrangement, so as to form a local resistance on the gas flow (50, 51) through said main channel (7, 27), whatever the flow direction thereof; and
- **in that** said local narrowing, which is in the form of a protruding internal ring, is longitudinally remote from and external to said oblong pressure area (Z), and
- **in that** said local narrowing (R2, R3) is arranged upstream from said oblong pressure area (Z), that is to say on the proximal side.

2. The artificial respiration device according to claim 1, **characterized in that** said local narrowing (R2) forms a narrowing of the internal section of the tubular element (25) and is an integral part of said device (D2).

3. The artificial respiration device according to claim 1, **characterized in that** it comprises a removable tubular body (40), in which is arranged said local narrowing (R3) forming a narrowing of the internal section of said tubular body.

4. The artificial respiration device according to one of the preceding claims, **characterized in that** it includes ambient air intake means (42) driven by gas circulating in said main channel (7, 27).

5. The artificial respiration device according to one of the preceding claims, **characterized in that** the protruding internal ring forming the local narrowing (R2, R3) includes obstacles (43) inhibiting the accidental introduction of an object liable to hermetically seal the tubular element or body.

6. The artificial respiration device according to claim 5, **characterized in that** the obstacles (43) are converging wings.

7. A laryngeal mask, **characterized in that** it comprises an artificial respiration device (D2) as specified according to one of previous claims.
